(19) Europäisches Patentamt
European Patent Office
Office européen des brevets

(11) **EP 2 471 379 A2**

(12) **EUROPEAN PATENT APPLICATION**

(43) Date of publication:
04.07.2012 Bulletin 2012/27

(21) Application number: 12162205.4

(22) Date of filing: 13.11.2007

(51) Int Cl.:
*A23K 1/18* (2006.01)   *A23K 1/16* (2006.01)
*A23K 1/14* (2006.01)   *A23K 1/10* (2006.01)
*A61K 36/28* (2006.01)   *A61K 36/48* (2006.01)
*A61K 36/064* (2006.01)   *A61K 38/02* (2006.01)

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.11.2006 GB 0622582**

(62) Document number(s) of the earlier application(s) in accordance with Art. 76 EPC:
**07824551.1 / 2 091 353**

(71) Applicant: **Biorigin Scandinavia AS**
**0253 Oslo (NO)**

(72) Inventor: **Elvebø, Odd**
**1475 Finstadjordet (NO)**

(74) Representative: **Gardner, Rebecca Katherine**
**Dehns**
**St Bride's House**
**10 Salisbury Square**
**London EC4Y 8JD (GB)**

Remarks:
This application was filed on 29-03-2012 as a divisional application to the application mentioned under INID code 62.

(54) **Treatment of ectoparasitic infestation**

(57) The present invention relates to glucan and/or mannan for use in treating an ectoparasitic infection or infestation in a fish, wherein Fabaceae protein is co-administered to said fish.

Salmon lice per 100 fish

Figure 1

**Description**

The present invention defines new methods for killing ectoparasites and/or preventing or inhibiting the adhesion of ectoparasites to an animal as well as the treatment and prevention of ectoparasite-related diseases and conditions and the prevention or reduction of infestation and infection of animals by ectoparasites.

The present invention is related to protecting fish or other animals, including humans, against pathogens or ectoparasites which can enter and/or attach to the body. Fish lice, as exemplified by the salmon lice, *Lepeophtheirus salmonis,* and *Caligus elongatus* is a serious problem for both wild and cultivated salmon. These parasites attach themselves to salmon and sea trout and feed off them, causing them serious distress; they multiply rapidly and are capable of spreading to other fish over large areas. Sea lice are common on adult salmon, but juvenile salmon are most badly affected. The ectoparasites inflict severe damage on the surface of the fish like big lesions, puncture wounds and open sores. In the end the fish dies or the general condition and quality of the fish does not qualify for sale. The outcome is substantial economic losses for the fish farming industry. Therefore.fish lice are considered to be one of the most important problems in the farming of salmonids, especially with regard to Atlantic salmon (*Salmo Salar*) and rainbow trout (*Oncorhynchus mykiss*) and other fish like, but not limited to, different species from the phylum *Chordata,* exemplified by the class *Actinoperygii* exemplified by different cod species (*Gadus sp.*).

The aquaculture industry has been using antihelminthics for treatment of ectoparasites. Bathing in formalin is a widespread treatment against many parasites, especially in fresh water, while bathing in organophosphates (metrifonate, dichlorvos, azamethiphos), pyrethroids (pyrethrum, cypermethrin, deltamethrin) or hydrogen peroxide are the most common bath treatments against e.g. sea lice (*Lepeophtheirus salmonis, Caligus elongatus*). Substances such as chitin synthesis inhibitors, diflubenzuron and teflubenzuron, ivermectin and emamectin are examples of orally-(feed)-administered substances. These agents given as injections, bath treatments or as infeed preparations can inflict severe damages to the environment and the ecosystem. These and other attempts to control this problem have had limited success, are costs-intensive and there also exist many practical operational constraints like toxicity for the user. Current approaches to minimising chemotherapeutant and antihelminthic use involve monitoring infestation levels and targeting treatment to when it is likely to be most effective. As a result, demand for an easy-to administrate, cost-effective, and environmentally friendly treatment is high.

With the present invention it is possible to reduce substantially the number of ectoparasites by adding a glucan and/or a mannan to a fish diet with a substantial amount of plant proteins. Preferably the glucan and/or mannan is part of a cell wall fraction or derived from cell walls. It has also been shown that it is possible to reduce substantially the number of ectoparasites by giving the fish or other target animal protein from a member of the *Asteraceae* family, without the need for a glucan and/or mannan component, although such a component further enhances the treatment of or protection from ectoparasitic infection.

In one aspect, the present invention provides *Asteraceae* protein for use in treating an ectoparasitic infection or infestation in an animal. Typically the protein is present in the form of a 'meal', a well known term in the art used to refer to the residue left after some or most of the oil from a plant, seed or bean etc. has been removed, e.g. in a crushing and solvent-extraction method. Preferably glucan and/or mannan are co-administered with the *Asteraceae* protein.

The *Asteraceae* is preferably from the genus *Helianthus,* most preferably it is *Helianthus annus* (sunflower).

The invention provides a method of treating an ectoparasitic infection in an animal which method comprises administration to said animal of an effective combination of.one or more plant proteins and glucan and/or mannan.

The invention further provides the use of glucan and/or mannan in the manufacture of a medicament for treating an ectoparasitic infection in an animal, wherein said medicament is administered to said animal as part of a dietary regimen which comprises one or more plant proteins.

In a further aspect is provided a product comprising (a) glucan and/or mannan and (b) a plant protein, for combined, separate or sequential administration to an animal for treating an ectoparasitic infection in said animal.

Another aspect of the present invention relates to veterinary compositions comprising a plant protein and glucan and/or mannan as well as articles and kits comprising these compositions as well as their use.

As used herein, unless otherwise clear from the context, 'treatment' includes prophylactic treatment, e.g. reducing or preventing initial infection or infestation by ectoparasites. Treatment of ectoparasitic infection or infestation may include killing the ectoparasites and/or preventing or inhibiting the adhesion of ectoparasites to an animal as well as the treatment and prevention of ectoparasite related diseases and conditions in an infected animal. Thus the number or viability of ectoparasites associated with treated animals will preferably be reduced. As described further herein, 'treatment' includes oral and topical administration of the active ingredient.

In a further aspect the present inventors have demonstrated the beneficial effects on ectoparasitic infestation of adding sunflower meal to an animal feed containing other plant proteins such as soy.

In a further aspect, the invention provides the use of protein from a member of the *Asteraceae* family in the manufacture of a medicament for treating ectoparasitic infection or infestation in an animal. Preferably the target animals are fish. Preferably infection or infestation is reduced by the above use. Preferably the protein is sunflower protein.

Preferably the animal also receives protein derived from legumes, e.g. from *Fabaceae,* preferably soy.

Various documents including, for example, publications and patents, are recited throughout this disclosure. All such documents are; in relevant part, hereby incorporated by reference. The citation of any given document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

Referenced herein are trade names for components including various ingredients utilized in the present invention. The inventors herein do not intend to be limited by materials under a certain trade name. Equivalent materials (e.g., those obtained from a different commercial source under a different name or reference number) to those referenced by trade name may be substituted and utilized in the descriptions herein.

The compositions herein may comprise, consist essentially of, or consist of any of the elements as described herein.

Preferably both a mannan and a glucan are used according to the invention.

Preferably there is synergy between the mannan and/or glucan and the *Asteraceae* protein in the prevention, inhibition and/or treatment of ectoparasitic infestations in animals, particularly fish.

Thus preferably in the methods and uses of the invention the glucan and/or mannan and the plant (e.g. *Asteraceae*) protein are present in the formulation (or in the dietary regimen) in synergistic proportions.

The term "synergy" with regard to the present invention, refers to the interaction of two or more agents in a composition according to the present invention so that their combined effect is greater than the sum of their individual effects.

As previously mentioned, the plant protein which exhibits synergy with the glucan/mannan is preferably from the family *Asteraceae,* most preferably sunflower.

The compositions of the invention do not consist of just natural whole products, e.g. a plant which may have within it protein and glucan/mannan, they comprise processed components. The glucan or mannan are typically not plant derived nor from the same species as the plant protein, e.g. *Asteraceae* protein, component.

Where the *Asteraceae* protein is administered as part of an animal feed formulation, *Asteraceae* meal will typically comprise 2-50%, preferably 5-40%, more preferably 8-30% of the total feed formulation.

Preferably the glucan and/or mannan are present in the formulations or used as part of cell wall fractions.

The mannan and/or glucan used in accordance with the present invention can be form a variety of different sources. Important sources for these components are yeasts as exemplified by *Saccharomyces cerevisiae.* The yeast cell wall consists mainly of polysaccharides made up of three sugars, glucose, mannose, and N-acetylglucosamine. The mannose polysaccharides are linked to proteins to form a mannoprotein layer mainly localized at' the external surface. Since the predominant carbohydrate in these proteins is mannose, they are called manno-proteins. Mannan-oligosaccharides prevents the lectin from binding to its gut cell receptor and hence contributes to reducing lectin toxicity.

The common feature of glucans that are active immune-stimulants, and preferred for use according to the present invention, is the β-1,3-chain of glucose molecules. Such glucans are most often referred to as beta-glucans. However, beta-glucans are active immune-stimulants only if there are "branches" on the β-1,3-chain of glucose molecules. These "branches" are attached by β-1,6-linkages and may consist of single : glucose molecules, as in beta-glucans from mushrooms, or chains of glucose molecules, as in the beta-glucan present in the cell wall of bakers yeast. Such branched beta-glucans are called β-1,3/1,6-glucans. Among products which are correctly defined as β-1,3/1,6-glucans, there are great variations in frequency and length of the β-1,6-linked branches.

Depending upon yeast strain and type, the glucan constitutes up to 25 % of the yeast cell wall dry weight. During the process of isolating beta-glucan from yeast the outer layer of manno-protein is removed as well as most of the inner content of the cell, leaving a "ghost" particle, or Whole Glucan Particle, constituting the beta-glucan layer.

Brewers yeast differs in composition from bakers yeast because it is grown under anoxic conditions, resulting in a low level of beta-glucan in the cell walls. Other yeasts which provide a source for the mannan and/or glucan include *Candida* sp., *Hansenula* sp., *Histoplasma* sp., *Kloeckera* sp., *Kluyveromyces* sp., *Pichia* sp., *Rhodotorula* sp., *Saccharomyces* sp., *Schizophyllum* sp., *Schizosaccharomyces* sp., *Torula* sp. and *Torulopsis* sp..

Another source of mannan and/or glucan are mushrooms or fungi exemplified by those belonging to the classes *Mastigomycotina, Ascomycotina, Basidiomycotina,* and *Deuteromycotina* (imperfect fungi). Other suitable fungi include *Aspergillus* sp., *Penicillium* sp., *Sclerotinia* sp., and *Sclerotum* sp. They have the beta-1,3/1,6-glucans scleroglucan, lentinan and schizophyllan which are extracted from medicinal mushrooms, and are active immune-stimulants.

A third source of mannan and/or glucan are the members of the *Gramineae* (grasses), amongst the Angiosperms, where they are major components of endosperm walls of commercially important cereals such as oat, barley, rye, sorghum, rice and wheat. Apart from these, plants are not preferred sources.

A fourth source of mannan and/or glucan are algae, exemplified by the classes *Chlorophyceae, Charophyceae Euglenophyceae, Phaeophyceae, Bacillariophyceae, Chrysophyceae, Xanthophyceae, Pyrrophyceae* and *Rhodophyceae.* Laminarin is an example for a glucan product from sea-weed.

It is also possible to derive said mannan and/or glucan from the cell walls of Bacteria like *Alcaligenes* (*Achromobacteriaceae*), *Agrobacterium* and *Rhizobium* (*Rhizobacteriaceae*). Examples are *Alcaligenes faecalis, Agrobacterium radiobacter* and *A. rhizogenes, Rhizobium japonicum og R. trifolii, Streptococcus pneumoniae* as well as the *Cyanobacteriaceae Anabaena cylindrica.*

The mannan and/or glucan, i.e. the carbohydrate components, can be used in different forms, untreated or treated/processed. In the animal feed and farming industry the cells of organisms, most often yeast cells, are used, and fed directly to the animals. These products come in different forms and shapes, like compressed, liquid, crumbled, dry, active, in-active cells, and combinations like active dry, instant active dry and inactive dry. These products are most often the remnants of the cells used for other production processes like brewing or baking.

Thus the glucan/mannan component can be particulate or soluble or in any other physical state between a particulate and soluble product.

In addition to direct feeding of sources of mannan and/or glucan, the industries use processed products or cell extracts. These products may be hydrolysed or autolysed cells, partially or completely purified cell walls, isolated cell components other then the cell walls, isolated sources or a mixture of isolated cell or sources, either used as focused end result of the production process or by mixing these components to ready-to-use formulas. Another usual application is to mix the yeast cell or one or more yeast cell components with other ingredients after their extraction or simply use them together. All these products are available in various forms suited to different types of use: liquid, semi-paste, paste, fine powder, oil-coated powder, microgranulated powder, to mention only some.

Products containing isolated carbohydrate components may be combination products of two or more components (e.g. from the yeast cell wall), for example a combination of glucans and mannans.

The carbohydrate component may be mixed with other agents not being part of the cell walls, like vitamins or minerals. Examples of this group of products are mixtures of beta-glucans, mannose and peptidoglycans; glucan-products combined with minerals and vitamins as well as mixtures of beta-glucans, nucleotides, mannose, vitamins, minerals and other components.

Preferred glucans are those derived from yeast cell walls which have been treated by acid or enzyme to significantly reduce or eliminate (1,6) linkages within the glucan branches (a single (1,6) link is required to form the branch).). Thus, preferably less than 10%, more preferably less than 5%, most preferably less than 3% or 2% of the glycosidic bonds in the molecule will be (1,6) linkages.

Preferably the glucan component of a soluble glucan has a numerical average molecular weight of each single glucan chain from about 10 kDa to 30 kDa, preferably on average 20 kDa (+/- 5 kDa) and multiple chain MW in aqueous solution from abut 500 kDa to about 1500 kDa.

Preferred glucans of the invention have a beta-1,3 backbone, i.e. the backbone is made up of beta-1,3 linked glucopyranose units. These preferred glucans have one or more beta-1,3 side chains, i.e. side chains attached to the backbone via a beta-1,6 linkage and where the side chains are made up of beta-1,3 linked glucopyranose units. The side chain comprises 2 or more, typically 3, 4, 5, 10 or more beta-1,3 linked glucopyranose units.

Mannan is a polysaccharide containing a high proportion of mannose sub-units. Preferably it is made up of' D-mannose, D-glucose and D-galactose at a ratio of approximately 3:1:1.

A preferred source for the glucan and/or mannan are cell walls from *Saccharomyces cerevisiae.* A preferred source of glucan and/or mannan for use in the present invention is the yeast product PatoGard® as sold by Immunocorp, a Norwegian based company. The composition of said product is as follows:

| Component | weight % |
|---|---|
| Carbohydrate: | min 40 |
| Protein: | max 32 |
| Ash: | max 8 |
| Lipids: | max 15 |
| Moisture: | max 8 |

Typical values for the carbohydrate components are as follows:

| Component | % of total carbohydrates |
|---|---|
| Glucan | 20 |
| Mannan | 25 |

(continued)

| Component | % of total carbohydrates |
|---|---|
| Chitin | < 1 |
| Glycogen | < 2 |

The most preferred source for the glucan and/or mannan component for ectoparasitic applications are cell walls from *Saccharomyces cerevisiae.* Of these, the most preferred source is the hydrolyzed yeast product MacroGard® Feed Ingredient as sold by Immunocorp, a Norwegian based company. The composition of said product is as follows:

| **COMPOSITION** | **% by weight** | **typical range** |
|---|---|---|
| | | |
| CARBOHYDRATES | min 60 | 63-68 |
| LIPIDS | max 18 | 13-18 |
| PROTEIN | max 8 | 5-7 |
| ASH | max 12 | 6-10 |
| TOTAL SOLID | min 92 | 94-97 |

An alternative is MacroGard®Pet which has the following composition:

| *Component* | *% by weight* | *% Typical range* by weight |
|---|---|---|
| Carbohydrates | min 65 | 65-70 |
| Lipids | max 15 | 12-14 |
| Protein | max 8 | 5-7 |
| Ash | max 10 | 5-9 |
| Dry matter | min 92 | 94-97 |

A further source of glucan is MacroGard®AquaSol, which has the following composition:

| *Component* | *% of dry matter* | *Typical range* % |
|---|---|---|
| β-1,3/1,6-Glucan | min 95 | 96-99 |
| Lipids' | max 1 | 0-1.0 |
| Ash | max 2 | 0.1-1.0 |
| Protein | max 1 | 0-1.0 |

Other MacroGard® products include MacroGard® Immersion Grade, MacroGard® Adjuvant, and MacroGard® F1 Suspension. MacroGard® Feed Ingredient is particularly preferred.

PatoGard® and MacroGard® are suitable for all the methods and uses described herein.

A large number of plant protein sources may be used in connection with the present invention, particularly in embodiments where the ectoparasitic treatment is performed by presenting the *Asteraceae* protein as part of an animal, e.g. fish, feed. The main reason for using plant proteins in the animal feed industry is to replace more expensive protein sources, like animal protein sources. Another important factor is the danger of transmitting diseases thorough feeding animal proteins to animals of the same species. Examples for plant protein sources include, but are not limited to, protein from the plant family *Fabaceae* as exemplified by soybean and peanut, from the plant family *Brassiciaceae* as exemplified by canola, cottonseed, the plant family *Asteraceae* including, but not limited to sunflower, and the plant family *Arecaceae* including copra. These protein sources, also commonly defined as oilseed proteins can be fed whole, but they are more commonly fed as a by-product after oils have been removed. Other plant protein sources include plant protein sources from the family *Poaceae,* also known as *Gramineae,* like cereals and grains especially corn, wheat and rice or other staple crops such as potato, cassava, and legumes (peas and beans), some milling by-products including germ meal or corn gluten meal, or distillery/brewery by-products. The most preferred proteins according to the present invention are soybean proteins and sunflower proteins from the plant families *Fabaceae* and *Asteraceae.*

In the fish farming industry the major fishmeal replacers with plant origin reportedly used, include, but are not limited to, soybean meal (SBM), maize gluten meal, Rapeseed/canola (*Brassica* sp.) meal, lupin (*Lupinus* sp. like the proteins in kernel meals of dehulled white (*Lupinus albus*), sweet (*L. angustifolius*) and yellow (*L. luteus*) lupins, Sunflower (*Helianthus annuus*) seed meal, crystalline amino acids; as well as pea meal (*Pisum sativum*), Cottonseed (*Gossypium* sp.) meal, Peanut (groundnut; *Arachis hypogaea*) meal and oilcake, soybean protein concentrate, corn (*Zea mays*) gluten meal and wheat (*Triticum aestivum*) gluten, Potato (*Solanum tuberosum* L.) protein concentrate as well as other plant feedstuffs like Moringa (*Moringa oleifera* Lam.) leaves, all in various concentrations and combinations.

The protein sources may be in the form of non-treated plant materials and treated and/or extracted plant proteins. As an example, heat treated soy products have high protein digestibility. Still, the upper inclusion level for full fat or defatted soy meal inclusion in diets for carnivorous fish is between an inclusion level of 20 to 30%, even if heat labile antinutrients are eliminated. In fish, soybean protein has shown that feeding fish with protein concentration inclusion levels over 30% causes intestinal damage and in general reduces growth performance in different fish species. In fact, most farmers are reluctant to use more than 10% plant proteins in the total diet due to these effects.

Table A below illustrates how plant protein sources are not pure protein. Preferred pure protein levels are up to 30%, typically up to 20%, preferably 5-25%. The inclusion level of the glucan and/or mannan-comprising source PatoGard® as used in the present invention was 2000 mg/kg diet and for MacroGard® 1000 mg/kg diet. Much higher levels of up to several times this amount, e.g. 2-10 times, may be used in the present invention. Thus, depending on the animal to be treated, their age and health, the mode of administration etc., different concentrations of mannan/glucan may be used.

The proportion of plant protein to other protein (e.g. fish protein) in the total feed or diet is 5:95 to 95:5, preferably 15:85 to 50:50, more preferably 25:75 to 45:55.

The above figures are particularly appropriate for feeding to fish. Mammalian diets according to the invention may include less plant protein, e.g. up to 25%, preferably 5-20%.

Many different types of plant products are marketed and used in the animal feed industry. As an example, soy protein products may be cooked full-fat soybeans, Expeller Extracted soybean meal (SBM), Solvent Extracted SBM, Dehulled SBM, Soy Protein Concentrates or Soy Isolates, to mention a few.

Table A - Typical compositions of fish meal and vegetable protein ingredients currently used in commercial feeds for fish (% of dry matter)' are as follows:

| Protein source | Protein | Oil | Starch | NSP | Sugars |
|---|---|---|---|---|---|
| Fish meal | 78 | 12 | - | - | - |
| Full fat soy | 42 | 21 | 3 | 18 | 11 |
| Hulled and defatted soy | 57 | 1 | 3 | 23 | 14 |
| Soy protein concentrate | 68 | 1 | 7 | 19 | 2 |
| Extracted sunflower | 40 | 3 | 2 | 33 | 5 |
| Corn gluten | 67 | 2 | 21 | 3 | 1 |
| Wheat gluten | 85 | 6 | 7 | - | - |
| NSP = non-starch polysaccharides | | | | | |

This table describes the amount of protein in the various "protein sources". As an example, fish meal has a protein content of 78%. Thus the amount of a protein source present is not the same as the actual protein content.

According to the present invention is provided a composition comprising an effective amount of a combination of one or more plant proteins (preferably from an *Asteraceae*) and glucan and/or mannan together with a pharmaceutically or veterinary acceptable diluent, excipent or carrier. Such compositions have the utilities described above and in particular have utility in the treatment, including prophylactic treatment, of ectoparasitic infection or infestation of an animal, particularly a fish.

Ectoparasites are in general defined as parasites that live on or in the skin but not within the body. Examples are, but not limited to, the class *Insecta* including *Exop terygota* (like lice), *Endop terygota* like *Siphonaptera* (like fleas), *Diptera* (like true flies), the class *Acarina* (like ticks), from the orders *Mesostigmata, Prostigmata* and *Sarcoptiformes.*

The compositions of the present invention can be used in a wide variety of applications to prevent and/or inhibit ectoparasitic infections in animals. The fish farming industry, including of wild fish species and/or aquarium species, is a suitable environment for use with regard to the treatment of ectoparasites such as fish lice. Thus farmed and managed fish for human consumption may be treated as may feeder fish and other marine organisms which form part of the human food chain. Aquarium and ornamental domestic fish may also be treated.

Ectoparasitic treatment is also useful in mammalian farming and in the treatment and prevention of human ectoparasites.

Human ectoparasites which may be treated in accordance with the present invention include common bedbugs, fleas, leeches, lice, mites and ticks. In such cases it may be appropriate to administer the formulation as a cream, spray or lotion directly to the skin or in the form of a shampoo, cream or rinse to the hair. Oral administration is also suitable.

The non-fish farming industry is well practiced in ways of combating ectoparasitic infestations, e.g., scab, Fly Strike, ticks and the like in sheep. This may be by dipping or spraying as well as in animal feeds. Sheep are particularly vulnerable but cows, pigs and horses also suffer from ectoparasitic infestations. Domestic pets and working or captive animals may also be treated.

Frequently used carriers or auxiliaries in the pharmaceutical and veterinary formulations of use according to the present invention include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. The pH and exact concentration of the various components of the composition are adjusted according to routine skills. The compositions for veterinary use are preferably prepared and administered in dose units. The term "dose units" and its grammatical equivalents as used herein refer to physically discrete units suitable as unitary dosages for fish, each unit containing a predetermined effective and potentiating amount of at least one of the two active ingredients calculated to produce the desired therapeutic effect in association with the required physiologically tolerable carrier, e.g., a diluent or a vehicle. Dosage levels of the active compounds comprised in the synergetic composition of the present invention may vary.

By "an effective amount" is meant an amount of a compound, in a combination of.the invention, effective to ameliorate the symptoms of, or ameliorate, treat, prevent, delay the onset of or inhibit the progression of an infection or disease. Ultimately, the attending veterinarian will decide the appropriate amount and dosage regimen. The "effective amount" of the active ingredients that may be combined with the carrier materials to produce a single dosage will vary depending upon the host treated and the particular mode of administration. It will be understood, however, that the specific dose level for any particular animal will depend upon a variety of factors including the activity of the specific formulation employed, the site of infection, the infecting pathogen, the age, body weight, general health, sex, diet, time of administration, route of administration, rate of excretion, the duration of the treatment, the nature of concurrent therapy (if any), the severity of the particular disease undergoing treatment, the manner of administration and the judgment of the prescribing veterinarian.

The compositions according to the invention may be presented in the form of an article or carrier such as a tablet, coated tablet, lozenges, troches, syrups or elixirs, liposomes, powder/talc or other solid, solution, emulsions, suspension, liquid, spray, gel, drops, aerosol, douche, ointment, foam, cream, gel, paste, microcapsules, controlled release formulation, sustained release formulation or any other article or carrier which may possible or useful in light of the, at any give point in time and intended, preferred mode of administration.

The composition of the present invention may be provided alone or in combination with other medicaments to provide an operative combination. It is intended to include any chemically compatible combination of pharmacologically-active agents, as long as the combination does not eliminate the activity of the composition of the invention.

It will be appreciated that the composition of the present invention may be administered as a ready-to-use combination product, or each part of the composition of the present invention, may be administered separately, sequentially or simultaneously. Therapy may be repeated intermittently while parasites are detectable or even when they are not detectable. It might be relevant to administer the components two weeks prior to the expected challenge and/or for several weeks after the challenge. Continuous use is also possible.

The compositions may include one, two or several different plant proteins and one or more glucans and/or one or more mannan moieties, preferably at least one glucan and at least one mannan. However, preferred formulations contain relatively pure glucan as found in the MacroGard products described herein, so the glucan to mannan ratio will preferably be greater than 5:1, more preferably greater than 10:1, most preferably greater than 20:1; e.g. 5:1 to 100:1, preferably 10:1 to 100:1. The glucan and mannan combined will typically make up no more than 8%, preferably no more than 5%, more preferably no more than 2% of the total formulation (e.g. the total feed formulation) or diet.

The veterinary compositions include those adapted for enteral including oral administration, external application, like tablets, powders, granules or pellets for admixture with feed stuffs.

The subject for use of the compositions according to this invention, can be a primate, or other mammal, such as but not limited to dog, cat, horse, cow, pig, turkey, goat, monkey, chicken, rat, mouse, and sheep; as well as avian species, and aquatic animals, preferably fish.

For the purpose of this specification it will be clearly understood that the word "comprising" means "including but not limited to", and that the word "comprises" have a corresponding meaning. Therefore the words "comprise", "comprises", and "comprising" are to be interpreted inclusively rather than exclusively.

As used herein and in the appended claims, the singular forms "a," "an," and "the" include plural reference unless the context clearly dictates otherwise.

Where reference is made herein to use of *Asteraceae* or other plant proteins, unless otherwise clear from the context, it should be understood that *Asteraceae* or other plant meal may be used. In a further aspect, other components of the *Asteraceae* meal than the protein part may be used in place of the protein in the various medical and veterinary uses and methods described herein.

Unless defined otherwise, all technical and scientific terms and any acronyms used herein have the same meanings as commonly understood by one of ordinary skill in the art in the field of the invention.

The invention will now be further described in the following Examples and with reference to the figure in which

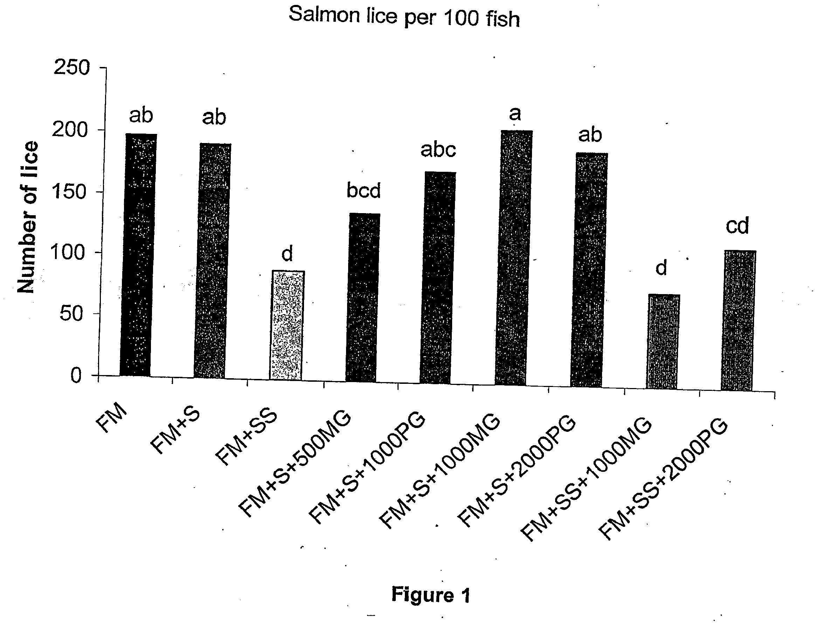

Figure 1 is a graph showing the number of lice per 100 Atlantic Salmon fed a range of diets according to the invention and control diets.

The following examples are intended to be illustrative of the present invention and to teach one of ordinary skill in the art to make and use the invention. These examples are not intended to limit the invention in any way.

EXAMPLES

1. Ingredients and diets

The formulation and composition of the diets is given in Tables 1 and 2, respectively. A standard fish meal based control diet (FM), a high-vegetable diet with 13.2% extracted and toasted soybean meal and 13.5% extracted sunflower meal [SFM] (FM+SS), and a high-vegetable diet with 29.9% soybean meal (FM+S) were manufactured by high-pressure moist extrusion by Skretting (Averøy, Norway). The particle size was 6 mm, and all diets were dried prior to coating with fish oil.

Prior to coating with oil, batches of the basis FM+SS diet was first coated with 1000 mg of MacroGard® (FM+SS+1000MG) or 2000 mg PatoGard® (FM+SS+2000PG) per kg diet. Likewise, batches of the basis FM+S diets was pre-coated 500 (FM+S+500MG) or 1000 (FM+S+1000MG) mg MacroGard® or 1000 (FM+S+1000PG) or 2000 (FM+S+2000PG) PatoGard® per kg diet. This gave a series of nine experimental diets.

Table 1. Formulation of the experimental diets.

| Diet code | FM-control | FM + SBM + SFM (SS) | FM + SBM (S) |
|---|---|---|---|
|  |  |  |  |
| Formula, g kg$^{-1}$ |  |  |  |
| LT-fish meal | 525.0 | 300.0 | 242.0 |
| SBM |  | 135.0 | 320.0 |
| SFM |  | 135.0 |  |
| Wheat gluten | 0.0 |  | 10.0 |
| Wheat | 188.0 | 116.5 | 100.5 |
| Fish oil | 286.0 | 291.0 | 305.0 |
| Lysine |  | 1.0 | 1.0 |
| Methionine |  | 1.5 | 1.5 |
| MCP* | 1.0 | 20.0 | 20.0 |
| *Mono Calcium Phosphate |  |  |  |

Table 2. Composition of the experimental diets

| Basic diet | FM | FM +S | FM + SS | FM + S | | FM + SS | | | |
|---|---|---|---|---|---|---|---|---|---|
| Added | MG | PG | MG | PG | MG | PG | | | |
| Dose, mg kg$^{-1}$ | 500 | 1000 | 1000 | 2000 | 1000 | 2000 | | | |
| | | | | | | | | | |
| Dry matter, g | 937.3 | 934.0 | 941.0 | 933.1 | 930.3 | 932.7 | 930.8 | 940.8 | 942.7 |
| Crude protein*, g | 385.0 | 348.5 | 340.9 | 347.3 | 346.0 | 351.0 | 345.8 | 341.2 | 359.5 |
| Lipid, g | 336.7 | 327.6 | 347.6 | 335.0 | 319.6 | 331.9 | 326.1 | 348.6 | 352.5 |
| Starch, g | 100.6 | 53.9 | 57.1 | 58.0 | 53.7 | 59.6 | 54.2 | 56.7 | 71.3 |
| Ash, g | 80.0 | 62.8 | 68.6 | 63.2 | 59.7 | 64.9 | 62.6 | 69.1 | 73.4 |
| Energy, MJ | 24.3 | 24.2 | 24.7 | 24.4 | 24.2 | 24.1 | 24.0 | 24.6 | 24.9 |
| *CP; N x 6.25 | | | | | | | | | |

2.2 Fish, rearing conditions, and sampling

Atlantic salmon (Salmo salar) were fed the experimental diets for a total of 69 to 71 feeding days. Prior to the experiment, the fish were fed commercial diets (Skretting AS, Stavanger, Norway). The experiment was initiated in week 25 and terminated in week 36 of 2006. The water temperature varied from 12.3 to 17.4 ˚C during the course of the experiment, averaging 15.3 ˚C.

At the start of the experiment, 27 groups of salmon (679 g, 150 fish per group) were randomly distributed to 5 x 5 x 5 m$^3$ sea pens. Each diet was then allocated to three groups of fish in a triplicate randomised experimental design. The fish were continuously fed by electrically driven feeders, and uneaten feed was collected from underneath the pens and pumped'up into wire mesh strainers as described by Einen (1999). The feeding rate was planned to be 15% in excess, and was adjusted according to the recorded overfeeding every three days as described by Helland et al. (1996).

The fish were weighed in bulk at the start of the experiment and on feeding day 70. At the final weighing a sufficient number of fish were also anesthetised with tricaine methanesulfonate (MS 222, Argent Chemical Laboratories Inc., Redmont, Wa, USA) and stripped as described by Austreng (1978) to collect faeces for digestibility estimation. The faecal samples were pooled per pen and immediately frozen at -20˚C.

Before the final weighing 20 fish per pen were weighed individually and sampled for counting of salmon and sea lice to evaluate degree of lice infestation. Following this, blood was collected from the caudal vein from of the fish 10 fish into heparinised vacutainers. Blood samples were kept on ice until centrifugation at 3000 rpm for 10 minutes. Plasma samples were aliquoted into eppendorf tubes, frozen on dry ice and stored at -20 ˚C.

Out of the 10 remaining fish, five fish were euthanised by a sharp cranial blow for sampling of tissue from the mid intestine, defined as the intestine from the most proximal to the most distal pyloric caeca, and distal intestine, defined as the region characterised by the transverse luminal folds and increased intestinal diameter to the anus. From each fish, 5 mm tissue samples were cut (a transverse cut relative to the length of the tract) from the central area of each intestinal section. These samples were placed and stored in phosphate-buffered formalin (4%, pH 7.2) for histological examination.

Before distributing fish to the experimental sea pen, 15 fish were sampled from the holding pen. These fish were euthanised in water with a lethal concentration of MS 222, weighed individually, and frozen immediately at -20˚C as three pooled samples of five fish. After feeding day 70 the fish were fasted for two days before this procedure was repeated, sampling five fish per pen. These pooled samples were ground frozen and homogenised for analyses of chemical composition.

2.3 Calculations

Crude protein (CP) was calculated as N x 6.25. Protein was estimated after hydrolysing the protein for amino acid analysis as the sum of dehydrated amino acids (as when peptide-bound). Thermal-unit growth coefficient (TGC) was calculated according to Iwama and Tautz (1981), modified by Cho (1992), as: TGC = $(W_1^{1/3} - W_0^{1/3}) \times (\Sigma D°)^{-1}$, where $W_0$ and $W_1$ are the initial and final weights (pen means), respectively, and $\Sigma D°$ is the thermal sum (feeding days x average temperature, ˚C). Feed intake was estimated by subtracting uneaten feed from fed feed on a dry matter basis.

Recovery of uneaten feed was estimated as described by Helland et al. (1996), and the recorded uneaten feed was corrected for dry matter losses during feeding and collection. Apparent digestibility was estimated by the indirect method, as described by Maynard and Loosli (1969), using $Y_2O_3$ as an inert marker (Austreng et al., 2000).

## 2.4 Chemical analyses and histological examination

Homogenised fish were freeze-dried (Hetosicc Freeze drier CD 13-2 HETO, Birkerød, Denmark) and analysed for dry matter (105˚C to constant weight), ash (combusted at 550˚C to constant weight), nitrogen (Kjeltec Auto Analyser, Tecator, Höganäs, Sweden), and lipid (pre-extraction with diethylether and hydrolysis with 4 M HCl prior to diethylether extraction (Stoldt, 1952) in a Soxtec (Tecator) hydrolysing (HT-6) and extraction (1047) apparatus).

Faeces were freeze-dried prior to analyses. Diets, and freeze dried faeces were analysed for dry matter, ash, nitrogen, lipid, starch (determined as glucose after hydrolysis by $\alpha$-amylase and amylo-glucosidase, followed by glucose determination by the "GODPOD method" (Megazyme, Bray, Ireland)), gross energy (Parr 1271 Bomb calorimeter, Parr, Moline, IL, USA) and yttrium (at Jordforsk, Ås, Norway, by inductivity coupled plasma (ICP) mass-spectroscopy, as previously described by Refstie et al. (1997)).

Formalin fixed intestinal tissue was routinely dehydrated in ethanol, equilibrated in xylene and embedded in paraffin according to standard histological techniques. Sections of approximately 5 $\mu$m were cut and stained with haematoxylin and eosin before examination under a light microscope. Intestinal morphology was evaluated according to the following criteria: (1) widening and shortening of the intestinal folds (2) loss of the supranuclear vacuolisation in the absorptive cells (enterocytes) in the intestinal epithelium; (3) widening of the central lamina propria within the intestinal folds, with increased amounts of connective tissue and (4) infiltration of a mixed leukocyte population in the lamina propria and submucosa. These are the characteristics of the condition previously described as soybean meal-induced enteritis in Atlantic salmon.

## 2.5 Statistical analyses

The results were analysed by the General Linear Model procedure in the SAS computer software (SAS, 1985). Mean results per pen were subjected to one-way analysis of variance (ANOVA) with diet as the independent variable. Significant differences were indicated by Duncan's multiple range test. The level of significance was P≤0.05, and the results are presented as mean $\pm$ s.e.m. (standard error of the mean).

## 3. Results and discussion

Applicable plant protein concentrations

Table 3 shows a calculation of the amount of plant protein in the total protein content of the animal feed diet 2 as used in the present invention.

| Protein Source | Protein content in commercially available animal feeds (in % of total) | Amount of protein source used in diet 2 (in % of the total feed) | Total protein content in the diet (in % of the total feed) | Protein content in % of the total protein content in the feed |
|---|---|---|---|---|
| Fish meal source | 78 | 29.9 | 23,3 | 63% |
| Soy protein source | 50 | 13.2 | 6,6 | 18% |
| Sunflower protein source | 40 | 13.5 | 5,4 | 15% |
| | | | | |
| Wheat protein source | 10 | 15 | 1,5 | 4% |
| Summarized protein content in the feed | | | 36,8 | 100% |
| Total share of plant protein content in the feed | | | | 37% |

**EP 2 471 379 A2**

Table 5 shows that 37% of the protein content of the total feed was from plant protein sources, while 63% was the usual fish meal protein source. This is a significant increase of possible additions of plant proteins to animal feed compared to the 10% limit as used in the farming industry and which is defined as a commercially acceptable figure. It is, without doubt possible to increase this concentration to a higher level by increasing the amount of the glucan- and/or mannan-comprising source.

Lice infestation

Table 4 shows different diets including the products MacroGard® and PatoGard® in relation to the number of lice infesting fish.
Diets without MacroGard® and PatoGard® but containing *Asteraceae* are also included.
Table 4. % of the fish infested by salmon lice (*Lepeophteirus salmonis*) and sea lice (*Caligus elongatus*), number of lice per infested fish, and estimated number of lice per 100 fish after feeding the experimental diets for 70 days (n=3).

| | Salmon lice | | | Sea lice | | |
|---|---|---|---|---|---|---|
| Diet | Infested Fish % | Lice per infested fish | Lice per 100 fish | Infested Fish % | Lice per infested Fish | Lice per 100 fish |
| FM | 77.3[ab] | 2.54[a] | 197[ab] | 36.4 | 1.06 | 39 |
| FM+S | 77.3[ab] | 2.42[ab] | 191[ab] | 22.7 | 1.12 | 26 |
| FM+SS | 60.6[c] | 1.48[d] | 89[d] | 25.8 | 1.25 | 32 |
| FM+S+500MG | 72.7[abc] | 1.86[bcd] | 136[bcd] | 13.6 | 1.13 | 17 |
| FM+S+1000PG | 77.3[ab] | 2.24[abc] | 173[abc] | 19.7 | 1.07 | 21 |
| FM+S+1000MG | 84.8[a] | 2.43[ab] | 208[a] | 24.2 | 1.00 | 24 |
| FM+S+2000PG | 84.8[a] | 2.25[abc] | 191[ab] | 21.2 | 1.11 | 26 |
| FM+SS+1000MG | 43.9[d] | 1.75[cd] | 77[d] | 19.7 | 1.30 | 24 |
| FM+SS+2000PG | 68.2[bc] | 1.70[cd] | 115[cd] | 16.7 | 1.18 | 20 |
| ANOVA | | | | | | |
| P | <0.0001 | 0.0056 | 0.0009 | 0.2888 | 0.5800 | 0.6094 |
| $\sqrt{MSE}$ | 7.9 | 0.32 | 35 | 9.7 | 0.18 | 13 |

Different superscripts abcd within column indicates significant differences as indicated by Duncan's Multiple Range Test (P<0.05).
ANOVA = Analysis of Variance
The infestation with salmon lice was significantly reduced in groups fed diets containing 15% sunflower meal. This indicated that dietary extracted sunflower meal actually reduced salmon lice infestation.
When comparing the FM+SS diets, the frequency of salmon lice infested fish was reduced when supplementing this diet with 1000 mg MacroGard® per kg.

References

Austreng, E., 1978. Digestibility determination in fish using chromic oxide marking and analysis of contents from different segments of the gastrointestinal tract. Aquaculture 13, 265-272.

Austreng, E., Storebakken, T., Thomassen, M.S., Refstie, S., Thomassen, Y., 2000. Evaluation of selected trivalent metal oxides as inert markers used to estimate apparent digestibility in salmonids. Aquaculture 188, 65-78.

Cho, C.Y., 1992. Feeding systems for rainbow trout and other salmonids with reference to current estimates of

**11**

energy and protein requirements. Aquaculture 100, 107-123.

Einen, O., Mørkøre, T., Rørå, A.M.B., Thomassen, M.S., 1999. Feed ration prior to slaughter - a potential tool for managing product quality of Atlantic salmon (Salmo salar). Aquaculture 178, 149-169.

Helland, S.J., Grisdale-Helland, B., Nerland, S., 1996. A simple method for the measurement of daily feed intake of groups of fish in tanks. Aquaculture 139, 157-163.

Iwama, G.K., Tautz, A.F., 1981. A simple growth model for salmonids in hatcheries. Can. J. Fish. Aquat. Sci. 38, 649-656.

Maynard, L.A., Loosli, J.K., 1969. Animal Nutrition, 6th edn. McGraw-Hill, New York, NY, USA.

Refstie, S., Helland, S., Storebakken, T., 1997. Adaptation to soybean meal in diets for rainbow trout, Oncorhynchus mykiss. Aquaculture 153, 263-272.

SAS, 1985. SAS/STAT® Guide for personal computers, Version 6 Ed. Cary, N.C., SAS Institute Inc., 378 pp.

Stoldt, W., 1952. Vorschlag zur Vereinheitlichung der Fettbestimmung in Lebensmitteln. Fette, Seifen, Anstrichm. 54, 206-207.

Embodiments of the invention are also described in the claims of the International application as filed:

1. *Asteraceae* protein for use in treating an ectoparasitic infection.or infestation in an animal.

2. *Asteraceae* protein as claimed in claim 1 wherein said *Asteraceae* is a *Helianthus.*

3. *Asteraceae* protein as claimed in claim 1 or 2 wherein said protein is in the form of meal.

4. *Asteraceae* protein as claimed in any preceding claim wherein said animal is a human.

5. *Asteraceae* protein as claimed in any one of claims 1 to 4 wherein said animal is a non-human animal.

6. *Asteraceae* protein as claimed in claim 5 wherein said animal is a fish or other marine organism.

7. *Asteraceae* protein as claimed in any preceding claim wherein said ectoparasite is a lice.

8. *Asteraceae* protein as claimed in any preceding claim wherein glucan or mannan is co-administered to said animal.

9. *Asteraceae* protein as claimed in claim 8 wherein said glucan is a glucan with a backbone made of 1,3 linked glucopyranose sub-units.

10. *Asteraceae* protein as claimed in claim 9 which comprises branches of 1,3 linked-glucopyranose sub-units attached to the backbone by a 1,6 linkage.

11. *Asteraceae* protein as claimed in any one of claims 8 to 10 wherein said glucan is derived from *Saccharomyces cerevisiae.*

12. A method of treating an ectoparasitic infection or infestation in an animal which comprises administering to an animal in need thereof an effective amount of *Asteraceae* protein.

13. A method as claimed in claim 12 wherein glucan is co-administered to said animal.

14. Glucan or mannan for use in treating an ectoparasitic infection or infestation in an animal wherein *Fabaceae* protein is co-administered to said animal.

15. Glucan or mannan as claimed in claim 14 which is formulated together with said *Fabaceae* protein.

**EP 2 471 379 A2**

16. Glucan or mannan as claimed in claim 15 or 15 wherein said glucan or mannan is derived from *Saccharomyces cerevisiae.*

17. A product comprising (a) glucan and/or mannan and (b) *Asteraceae* protein, as a combined preparation for simultaneous, separate or sequential administration to an animal for treating an ectoparasitic infection in said animal.

18. *Asteraceae* meal or any component thereof for use in treating an ectoparasitic infection or infestation in an animal.

**Claims**

1. Glucan or mannan for use in treating an ectoparasitic infection or infestation in a fish, wherein *Fabaceae* protein is co-administered to said fish.

2. The glucan or mannan for use according to claim 1, wherein said glucan or mannan is formulated together with said *Fabaceae* protein.

3. The glucan or mannan for use according to claim 1 or claim 2, wherein said *Fabaceae* protein is soy.

4. The glucan or mannan for use according to any one of claims 1 to 3, wherein said *Fabaceae* protein is in the form of meal.

5. The glucan or mannan for use according to any one of claims 1 to 4, wherein said ectoparasite is a louse.

6. The glucan or mannan for use according to any one of claims 1 to 5, wherein said glucan is a glucan with a backbone made of β-(1,3)-linked glucopyranose sub-units.

7. The glucan or mannan for use according to claim 6, wherein said glucan comprises branches of β-(1,3)-linked glucopyranose sub-units attached to the backbone by a β-(1,6)-linkage.

8. The glucan or mannan for use according to any one of claims 1 to 7, wherein said glucan is derived from yeast.

9. The glucan or mannan for use according to claim 8, wherein said glucan is derived from *Saccharomyces cerevisiae.*

**13**

## Salmon lice per 100 fish

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **AUSTRENG, E.** Digestibility determination in fish using chromic oxide marking and analysis of contents from different segments of the gastrointestinal tract. *Aquaculture,* 1978, vol. 13, 265-272
- **AUSTRENG, E. ; STOREBAKKEN, T. ; THOMASSEN, M.S. ; REFSTIE, S. ; THOMASSEN, Y.** Evaluation of selected trivalent metal oxides as inert markers used to estimate apparent digestibility in salmonids. *Aquaculture,* 2000, vol. 188, 65-78
- **CHO, C.Y.** Feeding systems for rainbow trout and other salmonids with reference to current estimates of energy and protein requirements. *Aquaculture,* 1992, vol. 100, 107-123
- **EINEN, O. ; MØRKØRE, T. ; RØRÅ, A.M.B. ; THOMASSEN, M.S.** Feed ration prior to slaughter - a potential tool for managing product quality of Atlantic salmon (Salmo salar. *Aquaculture,* 1999, vol. 178, 149-169
- **HELLAND, S.J. ; GRISDALE-HELLAND, B. ; NERLAND, S.** A simple method for the measurement of daily feed intake of groups of fish in tanks. *Aquaculture,* 1996, vol. 139, 157-163
- **IWAMA, G.K. ; TAUTZ, A.F.** A simple growth model for salmonids in hatcheries. *Can. J. Fish. Aquat. Sci.,* 1981, vol. 38, 649-656
- **MAYNARD, L.A. ; LOOSLI, J.K.** Animal Nutrition. McGraw-Hill, 1969
- **REFSTIE, S. ; HELLAND, S. ; STOREBAKKEN, T.** Adaptation to soybean meal in diets for rainbow trout, Oncorhynchus mykiss. *Aquaculture,* 1997, vol. 153, 263-272
- **STOLDT, W.** Vorschlag zur Vereinheitlichung der Fettbestimmung in Lebensmitteln. Fette, Seifen. *Anstrichm.,* 1952, vol. 54, 206-207